# EUROPEAN PATENT SPECIFICATION

(11) **EP 1 786 885 B1**
(45) Date of publication and mention of the grant of the patent: **18.05.2011**
(21) Application number: 05797974.2
(22) Date of filing: 12.08.2005
(51) Int. Cl.: C09K 11/06, C07C 15/28

(54) **ELECTROLUMINESCENT MATERIALS AND DEVICES**
ELEKTROLUMINESZENZMATERIALIEN UND VORRICHTUNGEN
MATÉRIAUX ET DISPOSITIFS ÉLECTROLUMINESCENTS

(30) Priority: 12.08.2004 GB 0417927
(43) Date of publication of application: 23.05.2007
(73) Proprietor: Merck Patent GmbH, 64293 Darmstadt (DE)
(72) Inventor: KATHIRGAMANATHAN, Poopathy, North Harrow HA2 7NN (GB)
(74) Representative: Seemann, Joachim
(86) International application number: PCT/GB2005/003173
(87) International publication number: WO 2006/016176

(56) References cited:
- EP-A- 0 281 381
- EP-A- 1 182 183
- WO-A-2004/058783

## Description

The present invention relates to an electroluminescent composition, and to an electroluminescent device having a layer in which said composition is comprised.

Materials which emit light when an electric current is passed through them are well known and used in a wide range of display applications. Liquid crystal devices and devices which are based on inorganic semiconductor systems are widely used; however these suffer from the disadvantages of high energy consumption, high cost of manufacture, low quantum efficiency and the inability to make flat panel displays,

Organic polymers have been proposed as useful in electroluminescent devices, but it is not possible to obtain pure colours; they are expensive to make and have a relatively low efficiency.

Another compound which has been proposed is aluminium quinolate, but this requires dopants to be used to obtain a range of colours and has a relatively low efficiency.

Patent application WO98/58037 describes a range of lanthanide complexes which can be used in electroluminescent devices which have improved properties and give better results. Patent Applications PCT/GB98/01773, PCT/GB99/0367, PCT/GB99/04030, PCT/GB99/04024, PCT/GB99/04028, PCT/GB00/00268 describe electroluminescent complexes, structures and devices using rare earth chelates.

US Patent 5128587 discloses an electroluminescent device which consists of an organometallic complex of rare earth elements of the lanthanide series sandwiched between a transparent electrode of high work function and a second electrode of low work function with a hole conducing layer interposed between the electroluminescent layer and the transparent high work function electrode and an electron conducting layer interposed between the electroluminescent layer and the electron injecting low work function anode. The hole conducting layer and the electron conducting layer are required to improve the working and the efficiency of the device. The hole transporting layer serves to transport holes and to block the electrons, thus preventing electrons from moving into the electrode without recombining with holes. The recombination of carriers therefore mainly takes place in the emitter layer.

It is known that anthracene is an electroluminescent composition and can emit light in the blue region of the spectrum, but its performance has not been adequate to enable it to be used in electroluminescent devices.

EP-A-1182183 discloses anthracene derivatives having two anthracene rings linked at their 10-positions and having substituents e.g. styryl groups at their 9-positions. The compounds are disclosed for use in electroluminescent devices e.g. for providing recombination sites.

This invention relates to an electroluminescent composition comprising a host material and an anthracene dopant, as set out in claim 1 of the accompanying claims. It also relates to an electroluminescent device as defined in claim 5. 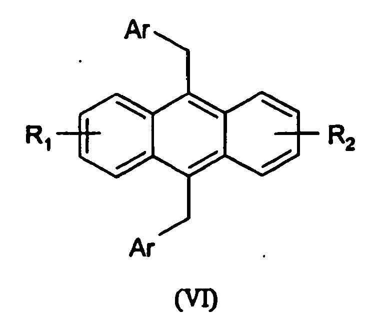 or 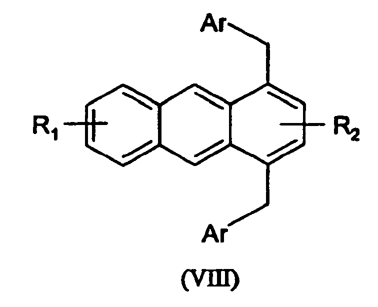 or
where Ar is an aromatic or a substituted aromatic group, and R₁ and R₂ are the same or different and are selected from hydrogen, substituted and unsubstituted aliphatic groups, substituted and unsubstituted aromatic, heterocyclic and polycyclic ring structures, fluorocarbons such as trifluoryl methyl groups, halogens such as fluorine or thiophenyl groups;
the host material has its upper band level higher than the upper band level of the dopant compound and its lower band level lower than the lower band level of the dopant compound being employed in an amount of 10⁻³ to 10 mole % of the host material.

The host material should have a bandgap which is wider than that of the aromatic substituted aromatic compound and the upper band level is higher than the upper band level of the aromatic substituted aromatic compound and the lower band level is lower than the lower band level of the aromatic substituted aromatic compound.

Preferably the host material is an electroluminescent compound.

As discussed in US 4769292, in theory, if the host material and the aromatic substituted anthracene could be found for blending which have exactly the same affinity for hole-electron recombination, each material should emit light upon injection of holes and electrons into the luminescent zone. The perceived hue of light emission would be the visual integration of both emissions.

Since imposing such a balance of the host material and aromatic substituted anthracene compounds is highly limiting, it is preferred to choose the aromatic substituted anthracene compound so that it provides the favoured sites for light emission. When only a small proportion of aromatic substituted anthracene compounds providing favoured sites for light emission are present, peak intensity wavelength emissions typical of the host material can be entirely eliminated in favour of a new peak intensity wavelength emission attributable to the aromatic substituted anthracene compound. While the minimum proportion of aromatic substituted anthracene compounds sufficient to achieve this effect varies by the specific choice of host material and aromatic substituted anthracene compounds, in no instance is it necessary to employ more than about 10 mole percent aromatic substituted anthracene compound based on moles of host material and seldom is it necessary to employ more than 1 mole percent of the aromatic substituted anthracene compound. On the other hand, for any host material capable of emitting light in the absence of aromatic substituted anthracene compounds, limiting the aromatic substituted anthracene compounds present to extremely small amounts, typically less than about 10⁻³ mole percent, based on host material, can result in retaining emission at wavelengths characteristic of the host material. Thus, by choosing the proportion of aromatic substituted anthracene compounds capable of providing favoured sites for light emission, either a full or partial shifting of emission wavelengths can be realized. This allows the spectral emissions of the EL devices of this invention to be selected and balanced to suit the application to be served.

Choosing aromatic substituted anthracene compounds capable of providing favoured sites for light emission, necessarily involves relating the properties of the aromatic substituted anthracene compounds to those of the host material. The host material can be viewed as a collector for injected holes and electrons with the aromatic substituted anthracene compounds providing the molecular sites for light emission. One important relationship for choosing aromatic substituted anthracene compounds capable of modifying the hue of light emission when present in a host material is a comparison of the reduction potentials of the two materials. The aromatic substituted anthracene compounds demonstrated to shift the wavelength of light emission have exhibited a less negative reduction potential than that of the host material. Reduction potentials, measured in electron volts, have been widely reported in the literature along with varied techniques for their measurement. Since it is a comparison of reduction potentials rather than their absolute values which is desired, it is apparent that any accepted technique for reduction potential measurement can be employed, provided both the fluorescent and host material reduction potentials are similarly measured. A preferred oxidation and reduction potential measurement technique is reported by R. J. Cox, Photographic Sensitivity, Academic Press, 773, Chapter 15.

A second important relationship for choosing aromatic substituted anthracene compounds capable of modifying the hue of light emission when present in a host material is a comparison of the bandgap potentials of the two materials. The aromatic substituted anthracene compounds demonstrated to shift the wavelength of light emission have exhibited a lower bandgap potential than that of the host material. The bandgap potential of a molecule is taken as the potential difference in electron volts (eV) separating its ground state and first singlet state. Bandgap potentials and techniques for their measurement have been widely reported in the literature. The bandgap potentials herein reported are those measured in electron volts (eV) at an absorption wavelength which is bathochromic to the absorption peak and of a magnitude one tenth that of the magnitude of the absorption peak. Since it is a comparison of bandgap potentials rather than their absolute values which is desired, it is apparent that any accepted technique for bandgap measurement can be employed, provided both the fluorescent and host material band gaps are similarly measured. One illustrative measurement technique is disclosed by F. Gutman and L. E. Lyons, Organic Semiconductors, Wiley, 767, Chapter 5.

Where a host material is chosen, which is itself capable of emitting light in the absence of the aromatic substituted anthracene compounds, it has been observed that suppression of light emission at the wavelengths of emission characteristics of the host material alone and enhancement of emission at wavelengths characteristic of the aromatic substituted anthracene compounds occurs when spectral coupling of the host material and aromatic substituted anthracene compounds is achieved. By spectral coupling it is meant that an overlap exists between the wavelengths of emission characteristic of the host material alone and the wavelengths of light absorption of the aromatic substituted anthracene compounds in the absence of the host material. Optimal spectral coupling occurs when the wavelength of maximum light emitted by said host material in the absence of said substituted anthracene compound is within 25 nm of the wavelength of maximum light absorption by said substituted anthracene compound alone. In practice advantageous spectral coupling can occur with peak emission and absorption wavelengths differing by up to 100 nm or more, depending on the width of the peaks and their hypsochromic and bathochromic slopes. Where less than optimum spectral coupling between the host material and aromatic substituted anthracene compounds is contemplated, a bathochromic as compared to a hypsochromic displacement of the aromatic substituted anthracene compounds produces more efficient results.

Although the foregoing discussion has been undertaken by reference to host materials which are known themselves to emit light in response to hole and electron injection, in fact light emission by the host material itself can entirely cease where light emission by the aromatic substituted anthracene compounds is favoured by any one or a combination of the various relationships noted above. It is appreciated that shifting the role of light emission to the aromatic substituted anthracene compounds allows a still broader range of choices of host materials. For example, one fundamental requirement of a material chosen to emit light is that it must exhibit a low extinction coefficient for light of the wavelength it emits to avoid internal absorption. The present invention permits use of host materials which are capable of sustaining the injection of holes and electrons, but are themselves incapable of efficiently emitting light.

Useful aromatic substituted anthracene compounds are those capable of being blended with the host material and fabricated into thin films satisfying the thickness ranges described above forming the luminescent zones of the electroluminescent devices of this invention. While crystalline host materials do not lend themselves to thin film formation, the limited amounts of aromatic substituted anthracene compounds present in the host material permits the use of aromatic substituted anthracene compounds which are alone incapable of thin film formation. Preferred aromatic substituted anthracene compounds are those which form a common phase with the host material.

Preferred host materials are conjugated aromatic compounds of formula :- 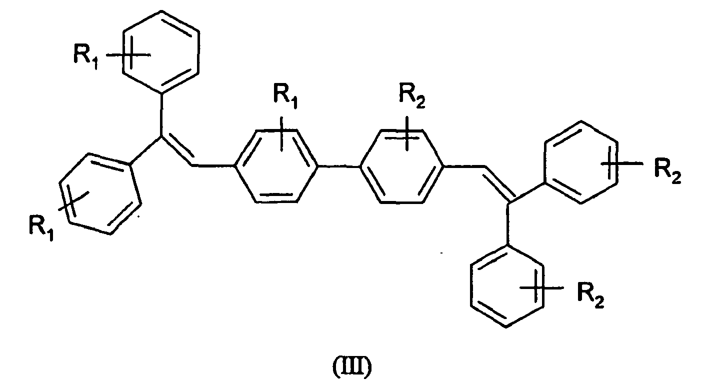 where R₁ and R₂ are as above;
or compounds of formula 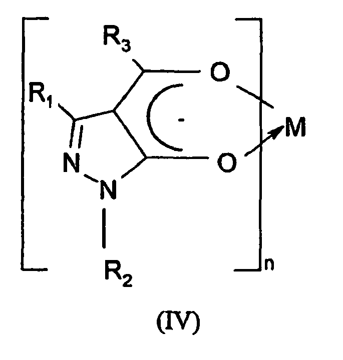 where M is a metal; n is the valency of M; R₁, R₂ and R₃ which may be the same or different are selected from hydrogen, hydrocarbyl groups, substituted and unsubstituted aliphatic groups substituted and unsubstituted aromatic, heterocyclic and polycyclic ring structures, fluorocarbons such as trifluoryl methyl groups, halogens such as fluorine or thiophenyl groups or nitrile; R₁, and R₃ can also be in the form of ring structures and R₁, R₂ and R₃ can be copolymerisable with a monomer, e.g. styrene;
or compounds of formula 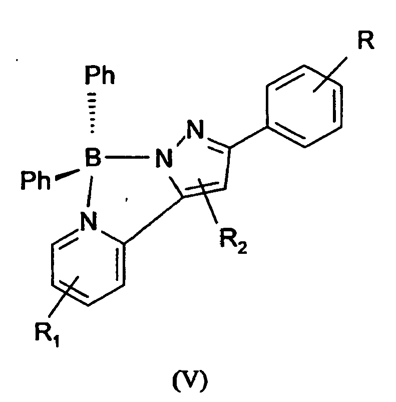 where Ph is an unsubstituted or substituted phenyl group where the substituents can be the same or different and are selected from hydrogen, and substituted and unsubstituted hydrocarbyl groups such as substituted and unsubstituted aliphatic groups, substituted and unsubstituted aromatic, heterocyclic and polycyclic ring structures, fluorocarbons such as trifluoryl methyl groups, halogens such as fluorine or thiophenyl groups; R, R₁ and R₂ can be hydrogen or substituted or unsubstituted hydrocarbyl groups, such as substituted and unsubstituted aromatic, heterocyclic and polycyclic ring structures, fluorine, fluorocarbons such as trifluoryl methyl groups, halogens such as fluorine or thiophenyl groups or nitrile.

Examples of R and/or R₁ and/or R₂ and/or R₃ include aliphatic, aromatic and heterocyclic alkoxy, aryloxy and carboxy groups, substituted and substituted phenyl, fluorophenyl, biphenyl, phenanthrene, anthracene, naphthyl and fluorene groups alkyl groups such as t-butyl, heterocyclic groups such as carbazole.

Preferably the layer of the electroluminescent composition is a thin film of less than 1 µm in thickness.

In the electroluminescent device of the invention the first electrode can function as the cathode and the second electrode can function as the anode and preferably there is a layer of a hole transporting material between the anode and the layer of the electroluminescent compound.

The hole transporting material can be any of the hole transporting materials used in electroluminescent devices.

The hole transporting material can be an amine complex such as poly (vinylcarbazole), N, N'-diphenyl-N, N'-bis (3-methylphenyl) -1,1' -biphenyl -4,4'-diamine (TPD), an unsubstituted or substituted polymer of an amino substituted aromatic compound, a polyaniline, substituted polyanilines, polythiophenes, substituted polythiophenes, polysilanes etc. Examples of polyanilines are polymers of 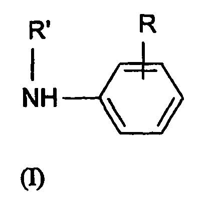 where R is in the ortho - or meta-position and is hydrogen, C1-18 alkyl, C1-6 alkoxy, amino, chloro, bromo, hydroxy or the group 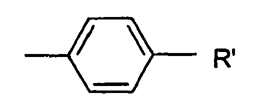 where R is alky or aryl and R' is hydrogen, C1-6 alkyl or aryl with at least one other monomer of formula (I) above.

Or the hole transporting material can be a polyaniline. Polyanilines which can be used in the present invention have the general formula 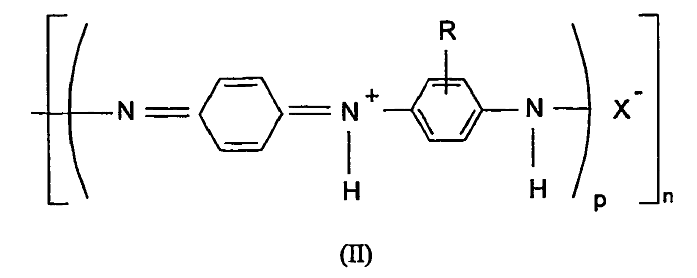 where p is from 1 to 10 and n is from 1 to 20, R is as defined above and X is an anion, preferably selected from Cl, Br, SO₄, BF₄, PF₆, H₂PO₃, H₂PO₄, arylsulphonate, arenedicarboxylate, polystyrenesulphonate, polyacrylate alkysulphonate, vinylsulphonate, vinylbenzene sulphonate, cellulose sulphonate, camphor sulphonates, cellulose sulphate or a perfluorinated polyanion.

Examples of arylsulphonates are p-toluenesulphonate, benzenesulphonate, 9,10-anthraquinone-sulphonate and anthracenesulphonate, an example of an arenedicarboxylate is phthalate and an example of arenecarboxylate is benzoate.

We have found that protonated polymers of the unsubstituted or substituted polymer of an amino substituted aromatic compound such as a polyaniline are difficult to evaporate or cannot be evaporated, however we have surprisingly found that if the unsubstituted or substituted polymer of an amino substituted aromatic compound is deprotonated then it can easily be evaporated i.e. the polymer is evaporable.

Preferably evaporable deprotonated polymers of unsubstituted or a substituted polymer of an amino substituted aromatic compound are used. The de-protonated unsubstituted or substituted polymer of an amino substituted aromatic compound can be formed by deprotonating the polymer by treatment with an alkali such as ammonium hydroxide or an alkali metal hydroxide such as sodium hydroxide or potassium hydroxide.

The degree of protonation can be controlled by forming a protonated polyaniline and de-protonating. Methods of preparing polyanilines are described in the article by A. G. MacDiarmid and A. F. Epstein, Faraday Discussions, Chem Soc.88 P37 789.

The conductivity of the polyaniline is dependant on the degree of protonation with the maximum conductivity being when the degree of protonation is between 40 and 60% e.g. about 50%.

Preferably the polymer is substantially fully deprotonated.

A polyaniline can be formed of octamer units i.e. p is four, e.g.

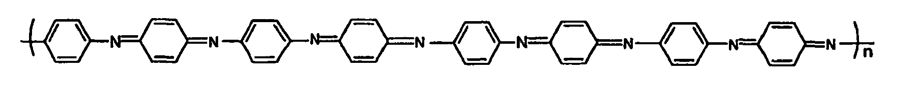

The polyanilines can have conductivities of the order of 1 x 10⁻¹ Siemen cm⁻¹ or higher.

The aromatic rings can be unsubstituted or substituted e.g. by a C1 to 20 alkyl group such as ethyl.

The polyaniline can be a copolymer of aniline and preferred copolymers are the copolymers of aniline with o-anisidine, m-sulphanilic acid or o-aminophenol, or o-toluidine with o-aminophenol, o-ethylaniline, o-phenylene diamine or with amino anthracenes.

Other polymers of an amino substituted aromatic compound which can be used include substituted or unsubstituted polyaminonapthalenes, polyaminoanthracenes, polyaminophenanthrenes, etc. and polymers of any other condensed polyaromatic compound. Polyaminoanthracenes and methods of making them are disclosed in US Patent 6,153,726. The aromatic rings can be unsubstituted or substituted e.g. by a group R as defined above.

Other hole transporting materials are conjugated polymers and the conjugated polymers which can be used can be any of the conjugated polymers disclosed or referred to in US 5807627, PCT/WO90/13148 and PCT/WO92/03490.

The preferred conjugated polymers are poly (p-phenylenevinylene)-PPV and copolymers including PPV. Other preferred polymers are poly(2,5 dialkoxyphenylene vinylene) such as poly (2-methoxy-5-(2-methoxypentyloxy-1,4-phenylene vinylene), poly(2-methoxypentyloxy)-1,4-phenylenevinylene), poly(2-methoxy-5-(2-dodecyloxy-1,4-phenylenevinylene) and other poly(2,5 dialkoxyphenylenevinylenes) with at least one of the alkoxy groups being a long chain solubilising alkoxy group, poly fluorenes and oligofluorenes, polyphenylenes and oligophenylenes, polyanthracenes and oligo anthracenes, ploythiophenes and oligothiophenes.

In PPV the phenylene ring may optionally carry one or more substituents e.g. each independently selected from alkyl, preferably methyl, alkoxy, preferably methoxy or ethoxy.

Any poly(arylenevinylene) including substituted derivatives thereof can be used and the phenylene ring in poly(p-phenylenevinylene) may be replaced by a fused ring system such as anthracene or naphthlyene ring and the number of vinylene groups in each polyphenylenevinylene moiety can be increased e.g. up to 7 or higher.

The conjugated polymers can be made by the methods disclosed in US 5807627, PCT/WO90/13148 and PCT/WO92/03490.

The thickness of the hole transporting layer is preferably 20nm to 200nm.

The polymers of an amino substituted aromatic compound such as polyanilines referred to above can also be used as buffer layers with or in conjunction with other hole transporting materials.

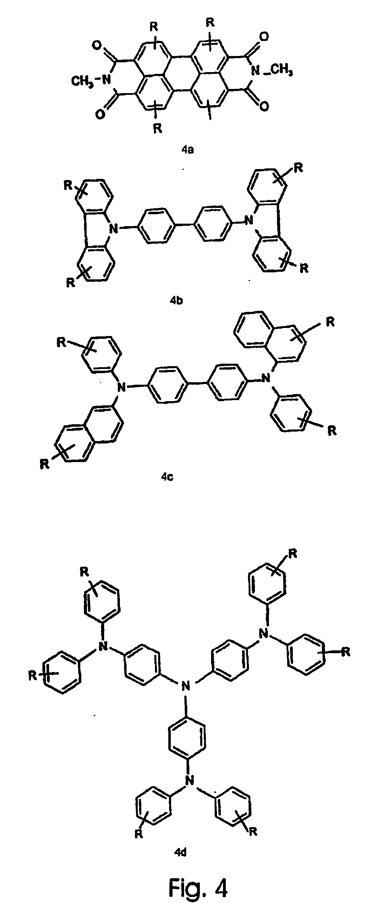

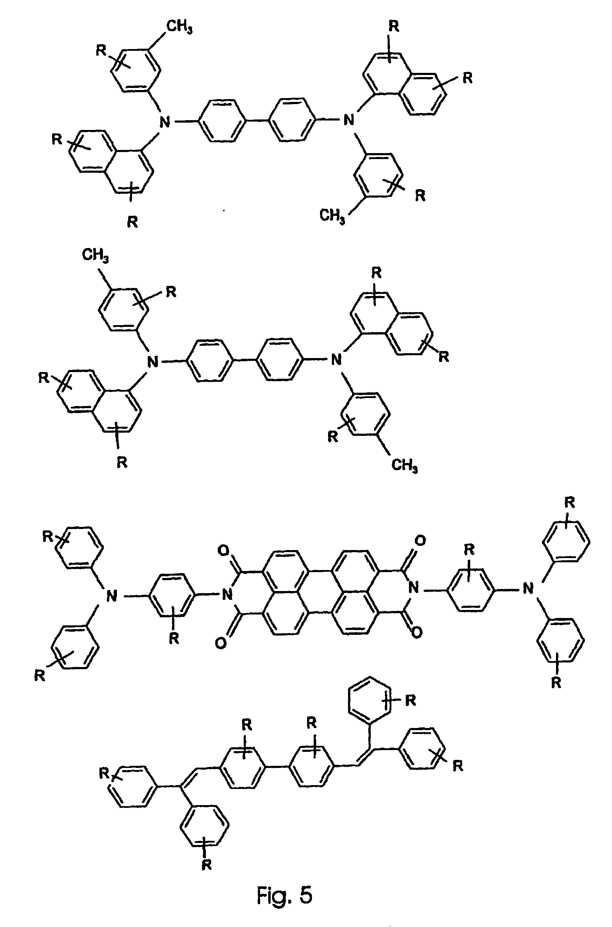

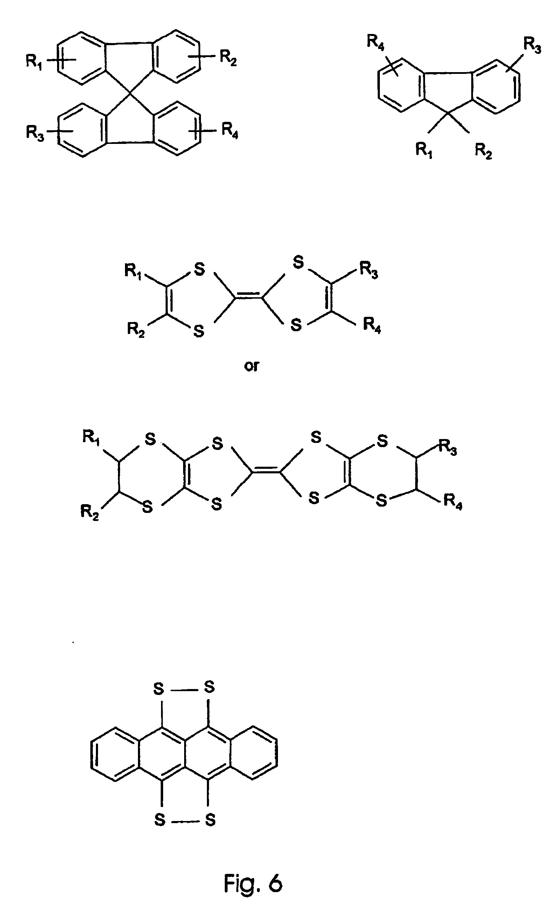

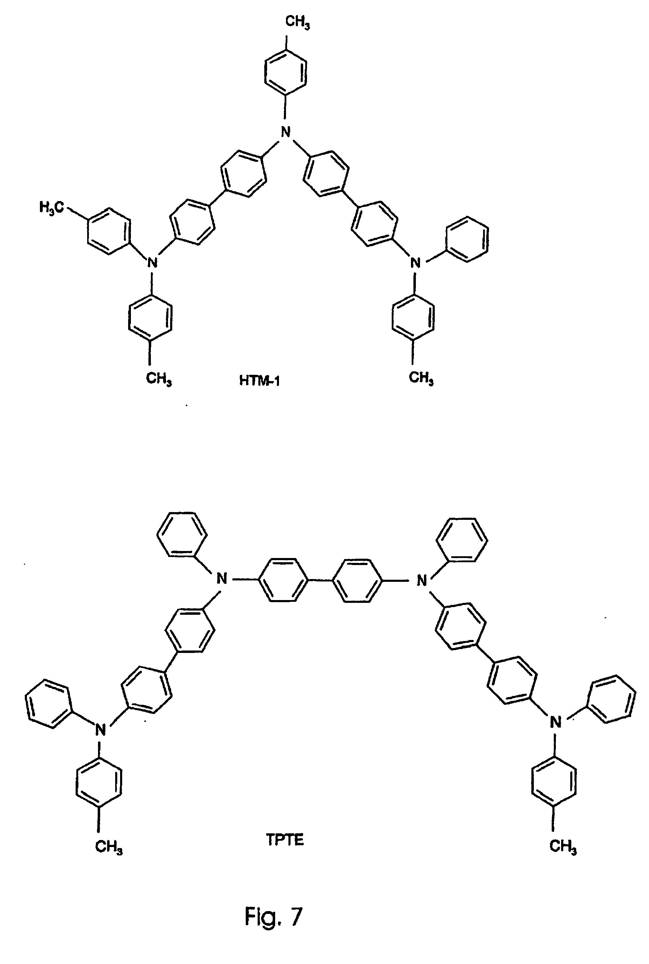

The structural formulae of some other hole transporting materials are shown in Figures 4, 5, 6, 7 and 8 of the drawings, where R₁, R₂ and R₃ can be the same or different and are selected from hydrogen, and substituted and unsubstituted hydrocarbyl groups such as substituted and unsubstituted aliphatic groups, substituted and unsubstituted aromatic, heterocyclic and polycyclic ring structures, fluorocarbons such as trifluoryl methyl groups, halogens such as fluorine or thiophenyl groups; R₁, R₂ and R₃ can also form substituted and unsubstituted fused aromatic, heterocyclic and polycyclic ring structures and can be copolymerisable with a monomer e.g. styrene. X is Se, S or O, Y can be hydrogen, substituted or unsubstituted hydrocarbyl groups, such as substituted and unsubstituted aromatic, heterocyclic and polycyclic ring structures, fluorine, fluorocarbons such as trifluoryl methyl groups, halogens such as fluorine or thiophenyl groups or nitrile.

Examples of R₁ and/or R₂ and/or R₃ include aliphatic, aromatic and heterocyclic alkoxy, aryloxy and carboxy groups, substituted and substituted phenyl, fluorophenyl, biphenyl, phenanthrene, anthracene, naphthyl and fluorene groups alkyl groups such as t-butyl, heterocyclic groups such as carbazole.

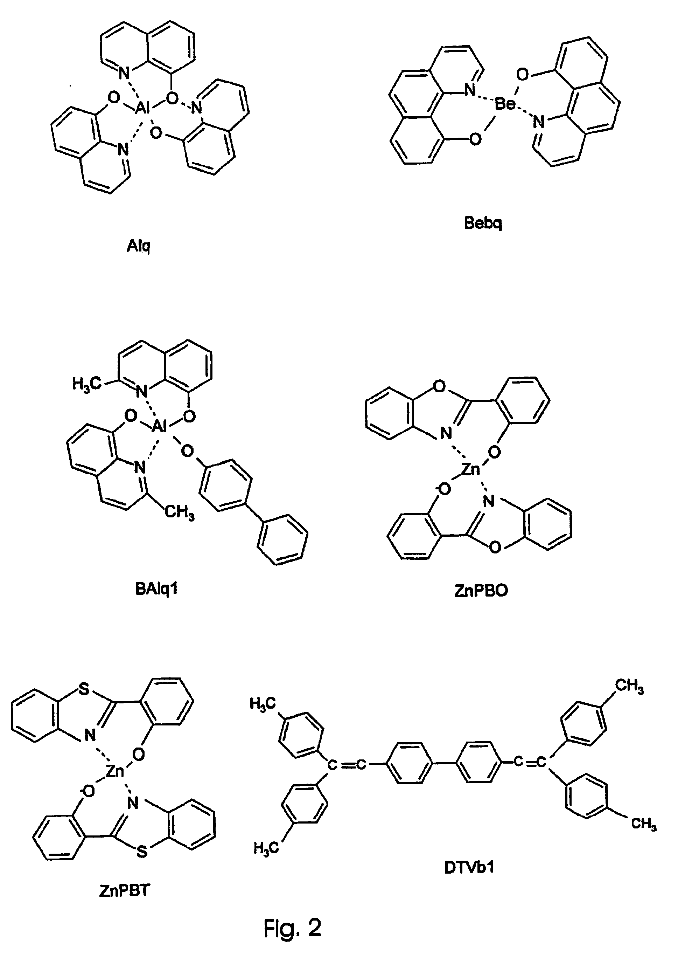

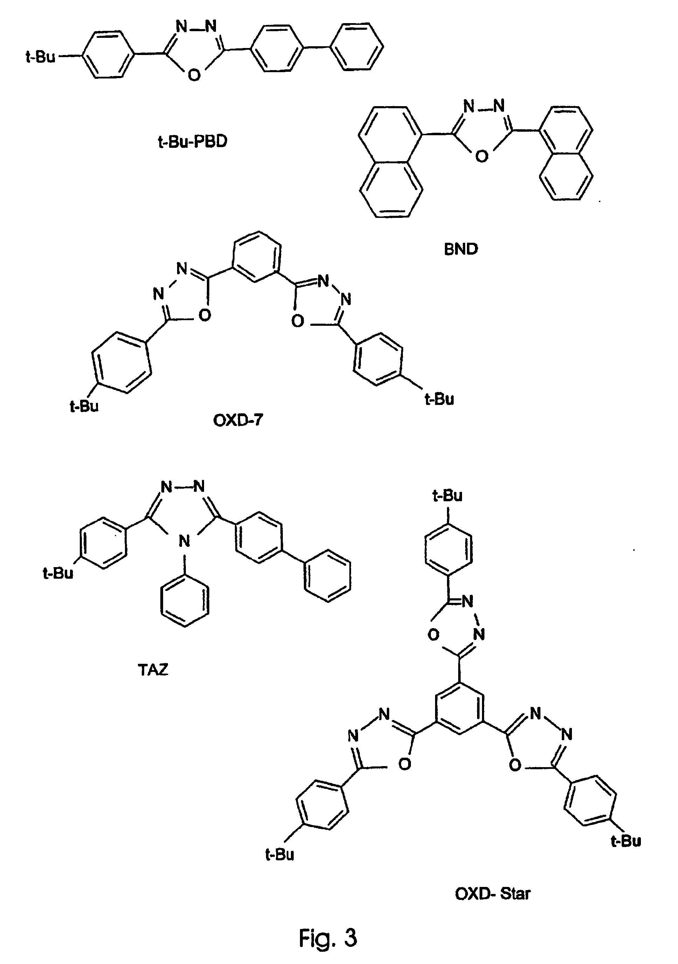

Optionally there is a layer of an electron injecting material between the cathode and the electroluminescent composition layer; the electron injecting material is a material which will transport electrons when an electric current is passed through electron injecting materials and include a metal complex such as a metal quinolate e.g. an aluminium quinolate, lithium quinolate, zirconium quinolate; a compound of formula Mx(DBM)ₙ where Mx is a metal and DBM is dibenzoyl methane and n is the valency of Mx, e.g. Mx is chromium. The electron injecting material can also be a cyano anthracene such as 9,10 dicyano anthracene, cyano substituted aromatic compounds, tetracyanoquinidodimethane a polystyrene sulphonate or a compound with the structural formulae shown in figures 2 or 3 of the drawings in which the phenyl rings can be substituted with substituents R as defined above; or a metal thioxinate of formula 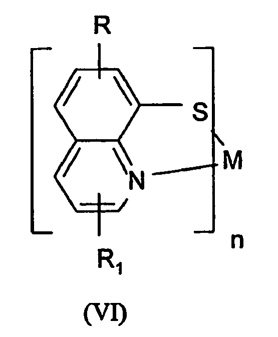 where M is a metal, preferably zinc, cadmium, gallium and indium; n is the valency of M; R and R₁ which can be the same or different are selected from hydrogen, and substituted and unsubstituted hydrocarbyl groups such as substituted and unsubstituted aliphatic groups, substituted and unsubstituted aromatic, heterocyclic and polycyclic ring structures, fluorocarbons such as trifluoryl methyl groups, halogens such as fluorine; thiophenyl groups; cyano group; substituted and unsubstituted hydrocarbyl groups such as substituted and unsubstituted aliphatic groups, substituted and unsubstituted aliphatic groups as described in patent application PCT/GB2005/002579.

The electron injecting material layer should have a thickness so that the holes form the anode and the electrons from the cathode combine in the electroluminescent layer. Instead of being a separate layer the electron injecting material can be mixed with the electroluminescent composition and co-deposited with it.

Optionally the hole transporting material can be mixed with the electroluminescent composition and co-deposited with it.

The hole transporting materials, the electroluminescent composition and the electron injecting materials can be mixed together to form one layer, which simplifies the construction.

The anode is preferably a transparent substrate such as a conductive glass or plastic material which acts as the anode. Preferred substrates are conductive glasses such as indium tin oxide coated glass, but any glass which is conductive or has a conductive layer such as a metal or conductive polymer can be used. Conductive polymers and conductive polymer coated glass or plastics materials can also be used as the substrate.

The cathode is preferably a low work function metal and preferably is comprised of a metal other than an alkali metal having a work function of less than 4 eV e.g. aluminium, calcium, lithium, magnesium and alloys thereof such as silver/magnesium alloys, rare earth metal alloys etc.; aluminium is a preferred metal. A metal fluoride such as an alkali metal, rare earth metal or their alloys can be used as the second electrode, for example by having a metal fluoride layer formed on a metal.

The invention is illustrated in the Examples in which examples 1-9 show the synthesis of the aromatic substituted anthracene compounds.

### Examples

### Synthesis for 9,10-Dibenzylanthracene Compounds

This is a general synthesis for these compounds. In each separate example a different benzyl halide compound is used, but the synthesis process is the same.

Anthracene (8.0g, 44.9mmol), Zinc dust (2.35g, 35.9mmol) and the benzyl chloride (94mmol) were stirred in carbon disulphide (150ml) and refluxed for 30h. The reaction was cooled to room temperature and the solvent was removed by distillation. The residue was extracted into hot toluene (200ml) and filtered under vacuum to remove excess zinc. On cooling. The toluene solution yielded a light yellow crystalline product which was recrystalised from hot toluene. filtered and dried in a vacuum oven.

### Example 1

For 9,10 Bis(4-methyl-benzyl)-anthracene (A) 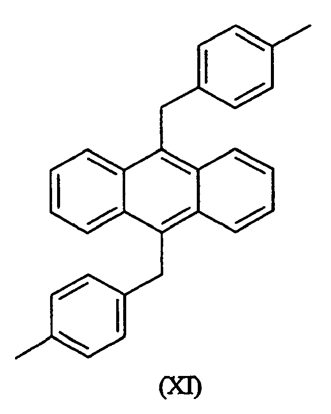 4-Methylbenzylchloride was used as the starting material

### Example 2

For 9,10-Bis-(2,4-dimethyl-benzyl)-anthracene (B) 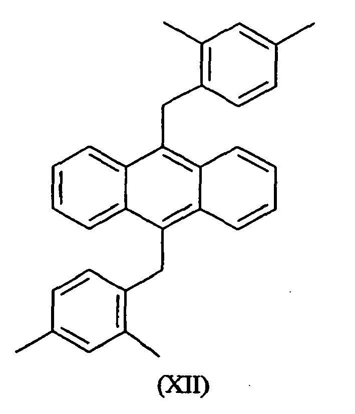 2,4-Dimethylbenzyl chloride was used as the starting material.

### Example 3

For 9,10-Bis-(2,5-dimethyl-benzyl)-anthracene (C) 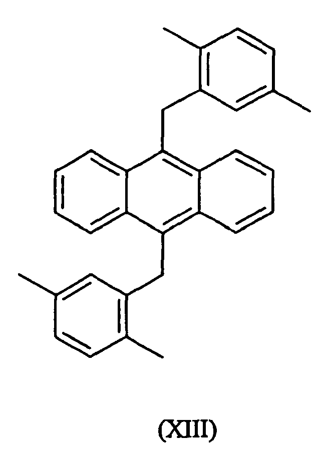 2,5-Dimethylbenzyl chloride was used as the starting material.

### Example 4

For 1,4-Bis-(2,3,5,6-tetramethyl-benzyl)-anthracene (D) 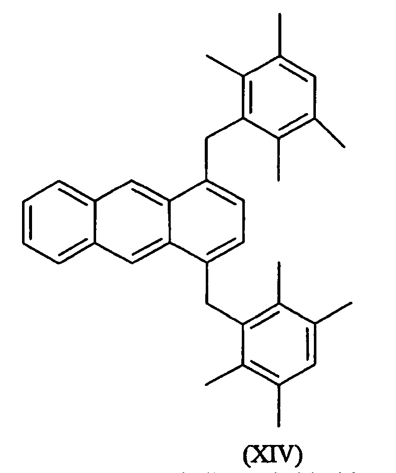 2,3,5,6-tetrameyhylbenzyl chloride was used as the starting material.

### Example 5

For 9,10-Bis-(4-methoxy-benzyl)-anthracene (E) 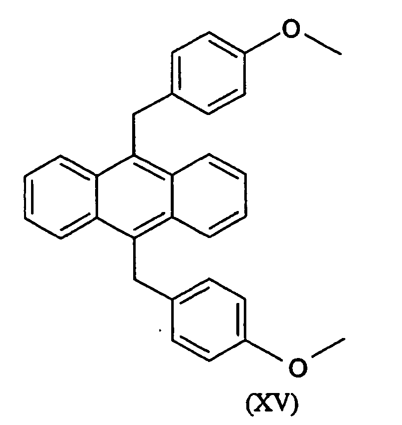 4-Methoxybenzyl chloride was used as the starting material.

### Example 6 (not according to the present invention)

For 9,10-Bis-(9H-fluoren-9-yl)-anthracene (F) 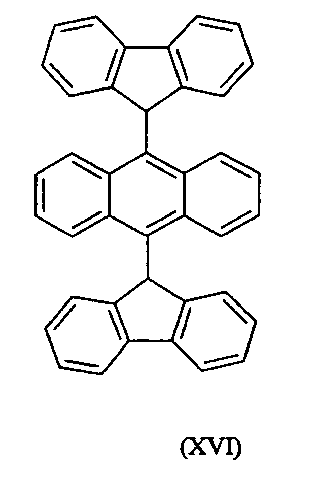 9-Bromofluorene was used as the starting material.

### Example 7 (not according to the present invention)

### Preparation of 2,6-Di-tert-butyl-anthracene

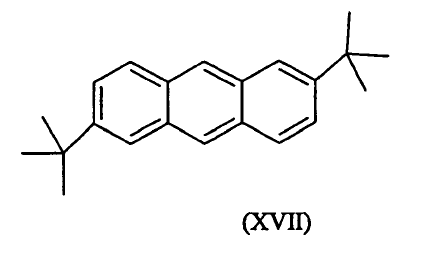

Anthracene (7.13g. 40mmol) and *tert-*Butanol (10.8g, 120mmol) were refluxed for 15h in trifluroacetic acid (40ml). The mixture was cooled and poured into water (250ml). The solid that formed was filtered under vacuum, washed with water and dried. The solid was recrystalised from hot hexane to yield a colourless crystalline solid. M.p. 249-253°C.

### Example 8

### Preparation of 2,6-Di-tert-butyl-9,10-bis-(2,5-dimethyl-benzyl)-anthracene (G)

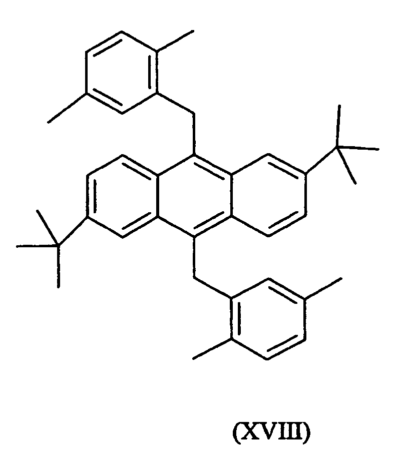

2,6-Di-tert-butyl-anthracene (3.0g, 10.3mmol), Zinc dust (0.54g, 8.3mmol) and 2,5-Dimethylbenzyl chloride (3.35g, 21.7mmol) were stirred in carbon disulphide (50ml) and refluxed for 30h. The reaction was cooled to room temperature and the solvent was removed by distillation. The residue was extracted into hot toluene (50ml) and filtered under vacuum to remove excess zinc. On cooling, the toluene solution yielded a colourless crystalline product which was recrystalised from hot toluene, filtered and dried in a vacuum oven. M.p. 273-275°C.

### Example 9

### Preparation of 2,6-Di-tert-butyl-9,10-bis-naphthalen-I -ylmethyl-anthracene (H)

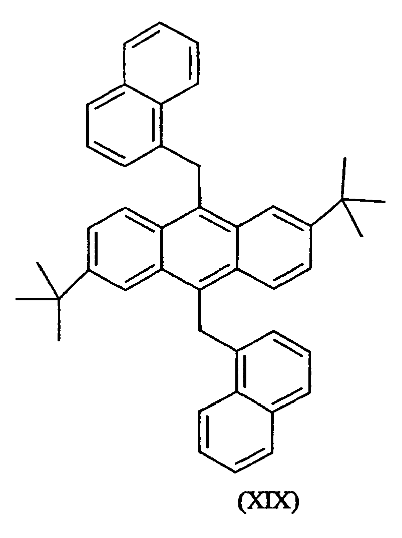

2,6-Di-tert-butyl-anthracene (2.9g, 10mmol), Zinc dust (0.52g, 8mmol) and 1-(chloromethyl)naphthalene (3.7g, 20.9mmol) were stirred in carbon disulphide (50ml) and refluxed for 30h. The reaction was cooled to room temperature and the solvent was removed by distillation. The residue was extracted into hot toluene (50ml) and filtered under vacuum to remove excess zinc. On cooling. The toluene solution yielded a colourless crystalline product which was recrystalised from hot toluene filtered and dried in a vacuum oven. M.p. 285°C.

### Electroluminescent Devices

### Example 10

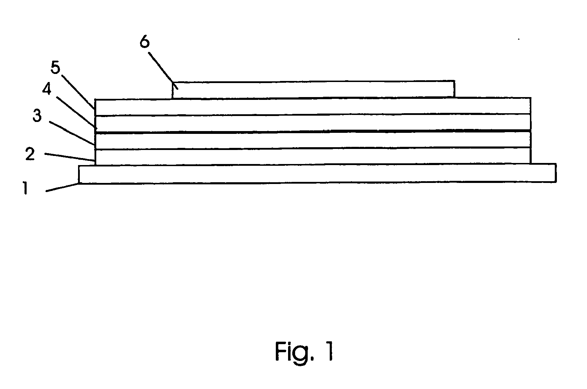

A pre-etched ITO coated glass piece (10 x 10cm²) was used. The device was fabricated by sequentially forming on the ITO, by vacuum evaporation the compositions forming the layers comprising the electroluminescent device. The layers were deposited using a Solciet Machine, ULVAC Ltd. Chigacki, Japan. The active area of each pixel was 3mm by 3mm, the device is shown in fig. 1 and the layers comprised:-
(1) ITO/ (2) α-NPB (42 nm)/ (3) K:H (20 : 1) (26 nm)/ (4) Zrq₄ (17 nm)/ (5) LiF (0.3 nm)/ (6) Al.

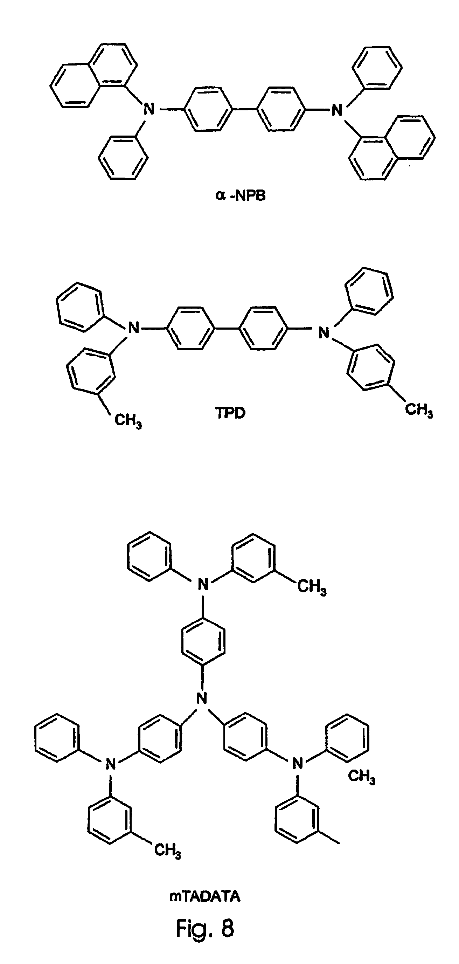

Where ITO is indium tin oxide coated glass, α-NPB is shown in the fig. 8 of the drawings, Zrq₄ is zirconium quinolate and K and H are as defined below.

The coated electrodes were stored in a vacuum desiccator over a molecular sieve and phosphorous pentoxide until they were loaded into a vacuum coater (Edwards, 10⁻⁶ torr) and aluminium top contacts made. The devices were then kept in a vacuum desiccator until the electroluminescence studies were performed.

The ITO electrode was always connected to the positive terminal. The current vs. voltage studies were carried out on a computer controlled Keithly 2400 source meter.

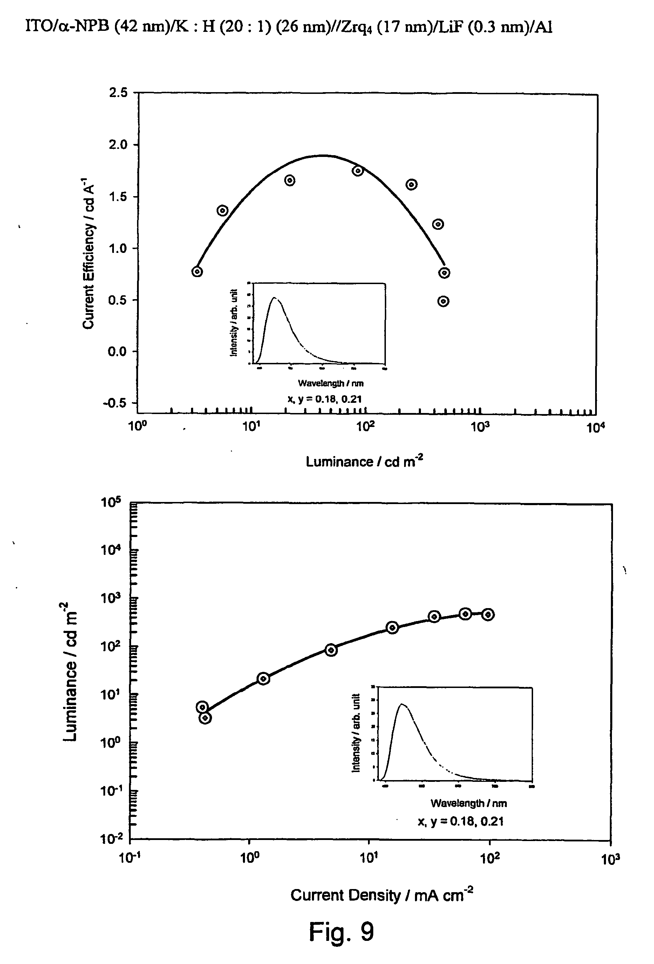

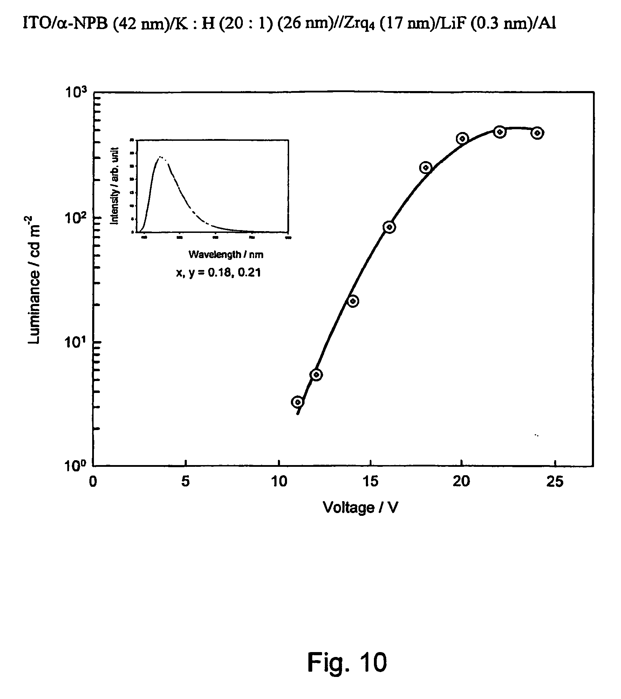

An electric current was applied across the device and the performance shown in figs. 9 and 10.

### Example 11

A device was formed as in example 10 with the structure:-
(1) TTO/α-NPB (40 nm)/(2) L : H (20 : 2) (27 nm)/(3) Zrq₄ (17 nm)/(4) LiF (0.3 nm)/ (5)Al.
where H and L are as shown below

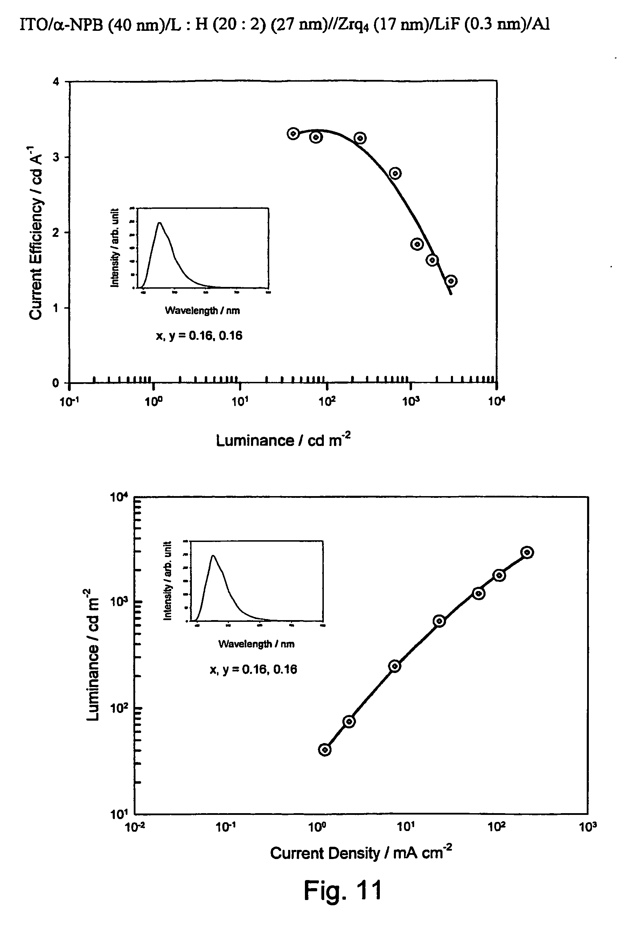

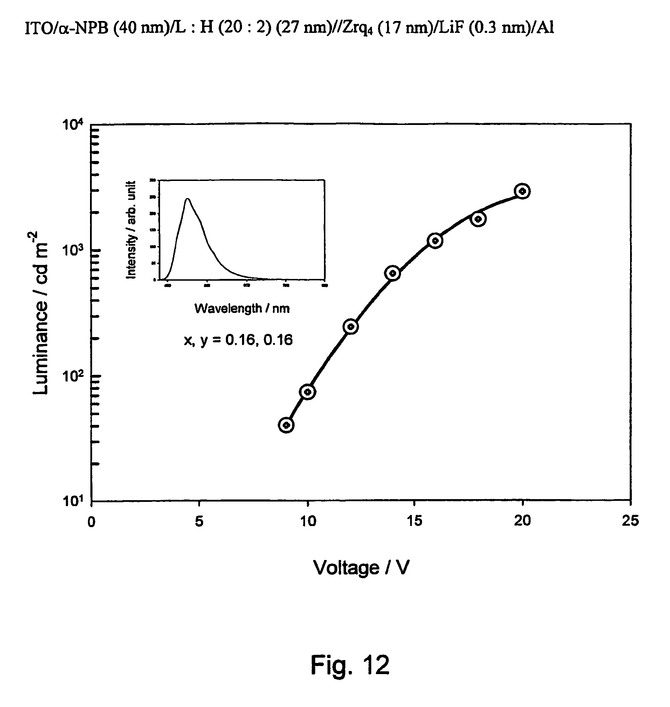

The performance is shown in figs. 11 and 12.

Compounds used in devices

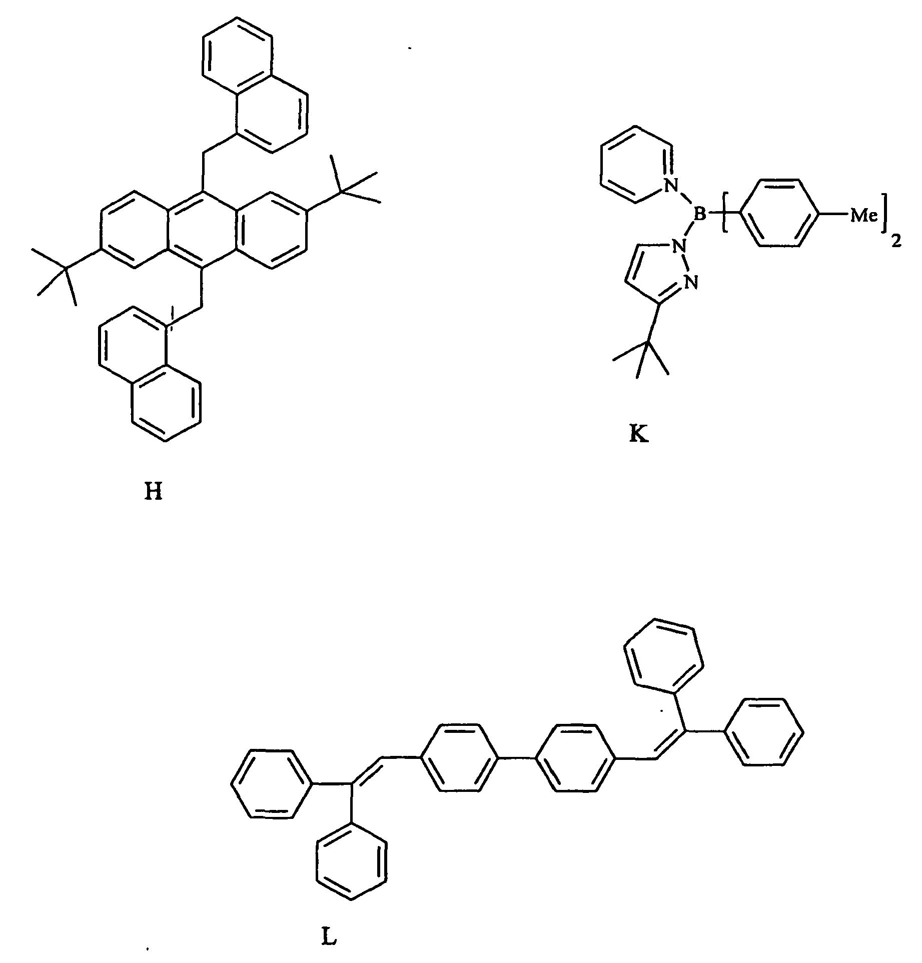

## Claims

1. An electroluminescent composition comprising a host material and a dopant which is a compound of any of the formulae: 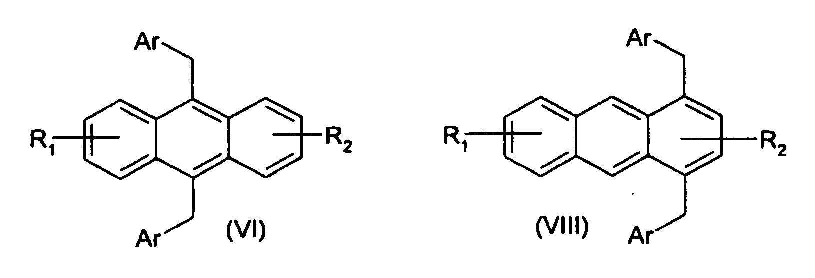 wherein
Ar is an aromatic or a substituted aromatic group; and
R₁ and R₂ which may be the same or different are selected from the group consisting of hydrogen, substituted or unsubstituted aliphatic groups, substituted and unsubstituted aromatic, heterocyclic and polycyclic ring structures, fluorocarbons, halogens and thiophenyl groups;
the host material has its upper band level higher than the upper band level of the dopant compound and its lower band level lower than the lower band level of the dopant compound being employed in an amount of 10⁻³ to 10 mole% of the host material.

2. The composition of claim 1, wherein the anthracene compound is selected from:
9,10-bis-(2,4-dimethylbenzyl)-anthracene;
9,10-bis-(2,5-dimethylbenzyl)-anthracene;
1,4-bis-(2,3,5,6-tetramethylbenzyl)-anthracene;
9,10-bis-(4-methoxybenzyl)-anthracene;
9,10-bis-(9*H*-fluoren-9-yl)-anthracene;
2,6-di-t-butyl-9,10-bis-(2,5-dimethylbenzyl)-anthracene; and
2,6-di-t-butyl-9,10-bis-naphthalen-1-ylmethyl-anthracene.

3. The composition of any preceding claim, wherein the host material is selected from the group consisting of aromatic compounds of formula: 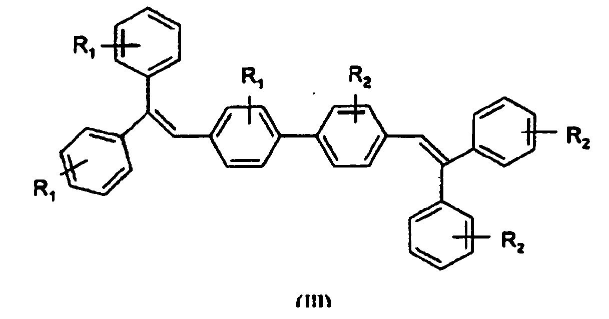 wherein R₁ and R₂ which may be the same or different are selected from the group consisting of hydrogen, substituted or unsubstituted aliphatic groups, substituted and unsubstituted aromatic, heterocyclic and polycyclic ring structures, fluorocarbons, halogens and thiophenyl groups, and
compounds of the formula 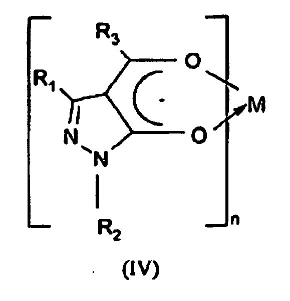 where
M is a metal;
n is the valence of M;
R₁, R₂ and R₃ which may be the same or different are selected from hydrogen, hydrocarbyl groups, substituted and unsubstituted aliphatic groups substituted and unsubstituted aromatic, heterocyclic and polycyclic ring structures, fluorocarbons, halogens such as fluorine or thiophenyl groups or nitrile; R₁ and R₃ can also be form ring structures and R₁, R₂ and R₃ can be copolymerisable with a monomer, e.g. styrene; and
compounds of the formula 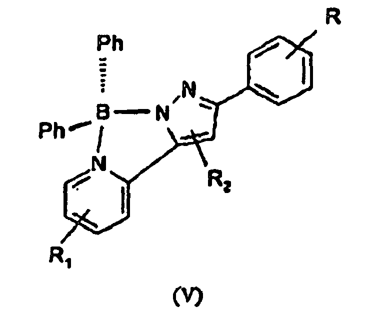 where Ph is an unsubstituted or substituted phenyl group where the substituents can be the same or different and are selected from hydrogen, substituted and unsubstituted aliphatic groups, substituted and unsubstituted aromatic, heterocyclic and polycyclic ring structures, fluorocarbon, halogens such as fluorine or thiophenyl groups; R, R₁ and R₂ can be hydrogen or substituted or unsubstituted aromatic, heterocyclic and polycyclic ring structures, fluorine, fluorocarbons, halogens such as fluorine or thiophenyl groups or nitrile.

4. The composition of claim 1 or 2, wherein the host comprises

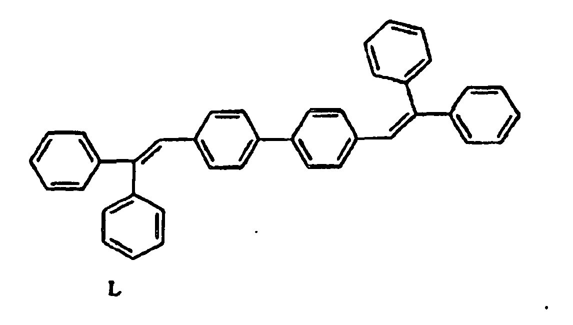

5. An electroluminescent device which comprises (i) a first electrode, (ii) a layer of electroluminescent composition as claimed in any one of claims 1 to 3 and (iii) a second electrode.

6. An electroluminescent device as claimed in claim 5 in which the layer of the electroluminescent composition is a film of less than 1 µm in thickness.

7. An electroluminescent device as claimed in claim 5 or 6 in which there is a layer of a hole transmitting material between the first electrode and the electroluminescent layer, the hole transmitting material being selected from the group consisting of:
α-NPB, TPD, mTADATA or another polyaromatic amine;
a film of a polymer selected from poly(vinylcarbazole), polyaniline, substituted polyanilines, polythiophenes, substituted polythiophenes, polysilanes and substituted polysilanes;
a copolymer of aniline, a copolymer of aniline with o- anisidine, m-sulphanilic acid or o-aminophenol, or o-toluidine with o-aminophenol, o-ethylaniline, o-phenylene diamine or with an amino anthracene; and
a conjugated polymer selected from poly (p-phenylenevinylene)-PPV and copolymers including PPV, poly(2,5 dialkoxyphenylene vinylene), poly (2-methoxy-5-(2- methoxypentyloxy-1,4-phenylene vinylene), poly(2-methoxypentyloxy)-1,4-phenylenevinylene), poly(2-methoxy-5-(2-dodecyloxy-1,4-phenylenevinylene) and other poly(2,5 dialkoxyphenylenevinylenes) with at least one of the alkoxy groups being a long chain solubilising alkoxy group, poly fluorenes and oligofluorenes, polyphenylenes and oligophenylenes, polyanthracenes and oligo anthracenes, ploythiophenes and oligothiophenes.

8. The device of any of claims 5-7, wherein there is a layer of an electron transmitting material between the cathode and the electroluminescent compound layer, the electron transmitting layer being selected from the group consisting of:
a metal quinolate or a metal thioxinate;
aluminium quinolate, zirconium quinolate or lithium quinolate, zinc thioxinate, cadmium thioxinate, gallium thioxinate or indium thioxinate;
a material of formula Mx(DBM)n where Mx is a metal and DBM is dibenzoyl methane and n is the valency of Mx; and
a cyano anthracene such as 9,10 dicyano anthracene, a polystyrene sulphonate; and
Bebq, ZnPBO, ZnPBT, DTVb1, BND, OXD-7, TAZ and OXD-Star.

## Patentansprüche

1. Elektrolumineszenzzusammensetzung, die ein Host-Material und einen Dotierstoff enthält, bei dem es sich um eine Verbindung einer beliebigen der Formeln: 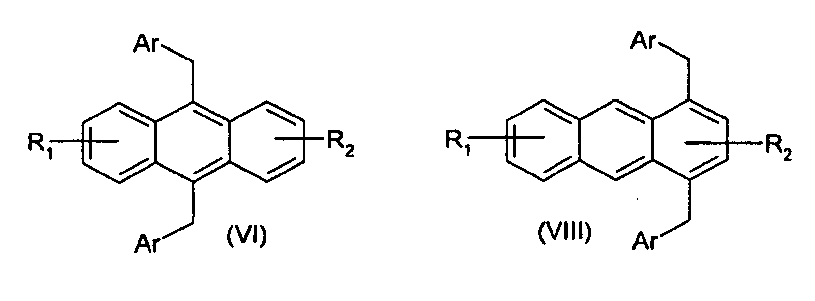 handelt, worin
Ar einen Aromaten oder eine substituierte aromatische Gruppe bedeutet; und
R₁ und R₂, die gleich oder verschieden sein können, aus der Gruppe bestehend aus Wasserstoff, substituierten oder unsubstituierten aliphatischen Gruppen, substituierten und unsubstituierten aromatischen, heterocyclischen und polycyclischen Ringstrukturen, Fluorkohlenwasserstoffen, Halogenen und Thiophenylgruppen ausgewählt sind;
die obere Bandlage des Host-Materials höher als die obere Bandlage der Dotierstoffverbindung und seine niedrigere Bandlage tiefer als die niedrigere Bandlage der Dotierstoffverbindung, die in einer Menge von 10⁻³ bis 10 Mol-% des Host-Materials eingesetzt wird, ist.

2. Zusammensetzung nach Anspruch 1, worin die Anthracenverbindung aus:
9,10-Bis-(2,4-dimethylbenzyl)-anthracen;
9,10-Bis-(2,5-dimethylbenzyl)-anthracen;
1,4-Bis-(2,3,5,6-tetramethylbenzyl)-anthracen;
9,10-Bis-(4-methoxybenzyl)-anthracen;
9,10-Bis-(9*H*-fluoren-9-yl)-anthracen;
2,6-Di-*t*-butyl-9,10-bis-(2,5-dimethylbenzyl)-anthracen und
2,6-Di-*t*-butyl-9,10-bis-naphthalin-1-ylmethyl-anthracen ausgewählt ist.

3. Zusammensetzung nach einem beliebigen vorhergehenden Anspruch, bei der das Host-Material aus der Gruppe bestehend aus aromatischen Verbindungen der Formel: 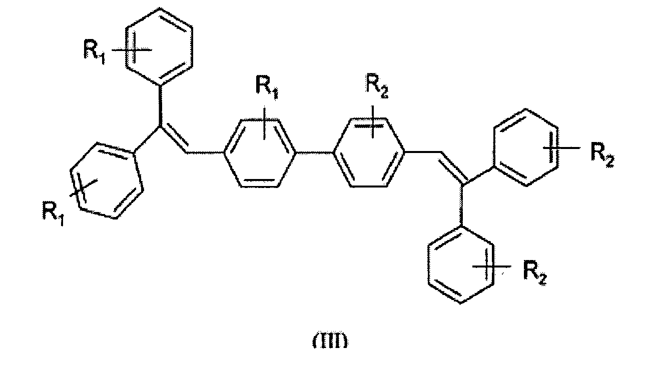 ausgewählt ist, worin R₁ und R₂, die gleich oder different sein können, aus der Gruppe bestehend aus Wasserstoff, substituierten oder unsubstituierten aliphatischen Gruppen, substituierten und unsubstituierten aromatischen, heterocyclischen und polycyclischen Ringstrukturen, Fluorkohlenwasserstoffen, Halogenen und Thiophenylgruppen ausgewählt sind, und
Verbindungen der Formel 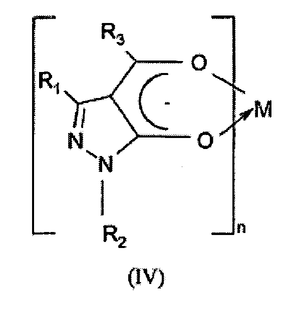 wobei
M ein Metall bedeutet;
n die Wertigkeit von M darstellt;
R₁, R₂ und R₃, die gleich oder verschieden sein können, aus Wasserstoff, Hydrocarbylgruppen, substituierten und unsubstituierten aliphatischen Gruppen, substituierten und unsubstituierten aromatischen, heterocyclischen und polycyclischen Ringstrukturen, Fluorkohlenwasserstoffen, Halogenen wie Fluor oder Thiophenylgruppen oder Nitril ausgewählt sind; R₁ und R₃ können auch Ringstrukturen bilden und R₁, R₂ und R₃ können mit einem Monomer, z.B. Styrol, copolymerisierbar sein; und
Verbindungen der Formel 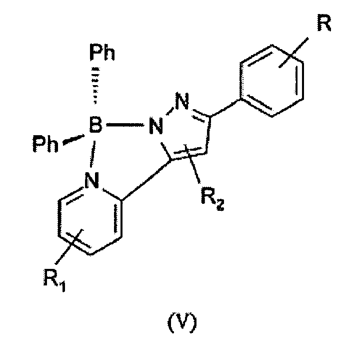 wobei Ph eine unsubstituierte oder substituierte Phenylgruppe bedeutet, wobei die Substituenten gleich oder verschieden sein können und aus Wasserstoff, substituierten und unsubstituierten aliphatischen Gruppen, substituierten und unsubstituierten aromatischen, heterocyclischen und polycyclischen Ringstrukturen, Fluorkohlenstoff, Halogenen wie Fluor oder Thiophenylgruppen ausgewählt sind und R, R₁ und R₂ Waserstoff oder substituierte oder unsubstituierte aromatische, heterocyclische und polycyclische Ringstrukturen, Fluor, Fluorkohlenwasserstoffe, Halogene wie Fluor oder Thiophenylgruppen oder Nitril bedeuten können,
ausgewählt ist.

4. Zusammensetzung nach Anspruch 1 oder 2, worin der Host 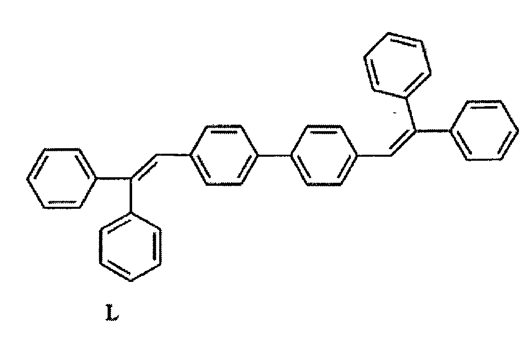 enthält.

5. Elektrolumineszenzvorrichtung, die (i) eine erste Elektrode, (ii) eine Schicht einer Elektrolumineszenzzusammensetzung nach einem beliebigen der Ansprüche 1 bis 3 und (iii) eine zweite Elektrode enthält.

6. Elektrolumineszenzvorrichtung nach Anspruch 5, in der die Schicht der Elektrolumineszenzzusammensetzung ein Film von weniger als 1 µm Dicke ist.

7. Elektrolumineszenzvorrichtung nach Anspruch 5 oder 6, in der sich zwischen der ersten Elektrode und der Elektrolumineszenzschicht eine Schicht eines Lochtransportmaterials befindet, welches aus der Gruppe bestehend aus:
α-NPB, TPD, mTADATA oder einem anderen polyaromatischen Amin;
einem Film eines Polymers, das aus Poly(vinylcarbazol), Polyanilin, substituierten Polyanilinen, Polythiophenen, substituierten Polythiophenen, Polysilanen und substituierten Polysilanen ausgewählt ist;
einem Copolymer des Anilins, einem Copolymer des Anilins mit o-Anisidin, m-Sulfanilsäure oder o-Aminophenol, oder des o-Toluidins mit o-Aminophenol, o-Ethylanilin, o-Phenylendiamin oder mit einem Aminoanthracen; und
einem konjugierten Polymer ausgewählt aus Poly(p-phenylenvinylen)-PPV und Copolymeren enthaltend PVV, Poly(2,5-dialkoxyphenylenvinylen), Poly(2-methoxy-5-(2-methoxypentyloxy-1,4-phenylenvinylen), Poly(2-methoxypentyloxy)-1,4-phenylenvinylen), Poly(2-methoxy-5-(2-dodecyloxy-1,4-phenylenvinylen) und anderen Poly(2,5-dialkoxyphenylenvinylenen), wobei es sich bei mindestens einer der Alkoxygruppen um eine langkettige löslichmachende Alkoxygruppe handelt, Polyfluorene und Oligofluorene, Polyphenylene und Oligophenylene, Polyanthracene und Oligoanthracene, Polythiophene und Oligothiophene
ausgewählt ist.

8. Vorrichtung nach einem beliebigen der Ansprüche 5-7, wobei sich zwischen der Kathode und der Schicht der Elektrolumineszenzverbindung eine Schicht eines Elektronentransportmaterials befindet, wobei die elektronentransportierende Schicht aus der Gruppe bestehend aus:
einem Metallchinolat oder einem Metallthioxinat;
Aluminium-, Zirconium- oder Lithiumchinolat, Zink-, Cadmium-, Gallium- oder Indiumthioxinat;
einem Material der Formel Mx(DBM)n, wobei Mx ein Metall bedeutet und DBM Dibenzoylmethan bedeutet und n die Wertigkeit von Mx darstellt; und
einem Cyanoanthracen wie 9,10-Dicyanoanthracen, einem Polystyrolsulfonat; und
Bebq, ZnPBO, ZnPBT, DTVb1, BND, OXD-7, TAZ und OXD-Star ausgewählt ist.

## Revendications

1. Composition électroluminescente comprenant un matériau hôte et un dopant qui est un composé de n'importe laquelle des formules : 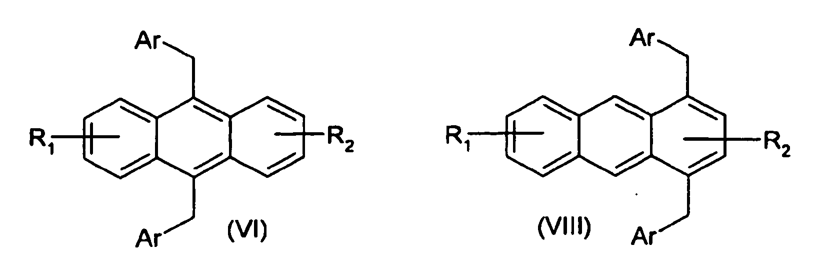 dans lesquelles
Ar est un aromatique ou un groupe aromatique substitué ; et
R₁ et R₂ qui peuvent être les mêmes ou différents sont choisis parmi le groupe constitué par hydrogène, des groupes aliphatiques substitués ou non substitués, des structures de cycle aromatiques, hétérocycliques et polycycliques substituées et non substituées, des fluorocarbones, des halogènes et des groupes thiophényle ;
le matériau hôte a son niveau de bande supérieure plus haut que le niveau de bande supérieure du composé de dopant et son niveau de bande inférieure plus bas que le niveau de bande inférieure du composé de dopant qui est utilisé selon une quantité de 10⁻³ à 10% mol. du matériau hôte.

2. Composition selon la revendication 1, dans laquelle le composé anthracène est choisi parmi :
9,10-bis-(2,4-diméthylbenzyl)-anthracène ;
9,10-bis-(2,5-diméthylbenzyl)-anthracène ;
1,4-bis-(2,3,5,6-tétraméthylbenzyl)-anthracène ;
9,10-bis-(4-méthoxybenzyl)-anthracène ;
9,10-bis-(9*H*-fluorène-9-yl)-anthracène ;
2,6-di-*t*-butyl-9,10-bis-(2,5-diméthylbenzyl)-anthracène ; et
2,6-di-*t*-butyl-9,10-bis-naphtalène-1-ylméthyl-anthracène.

3. Composition selon l'une quelconque des revendications précédentes, dans laquelle le matériau hôte est choisi parmi le groupe constitué par des composés aromatiques de formule : 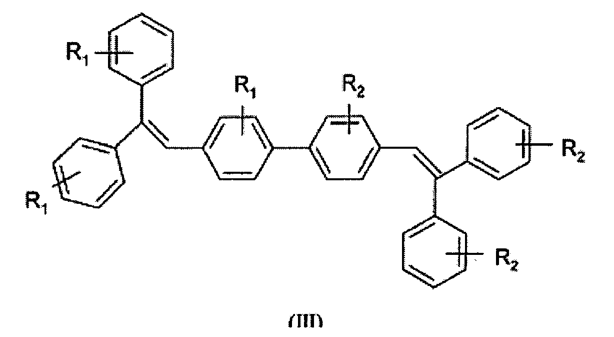 dans laquelle R₁ et R₂ qui peuvent être les mêmes ou différents sont choisis parmi le groupe constitué par hydrogène, des groupes aliphatiques substitués ou non substitués, des structures de cycle aromatiques, hétérocycliques et polycycliques substituées et non substituées, des fluorocarbones, des halogènes et des groupes thiophényle, et
des composés de la formule 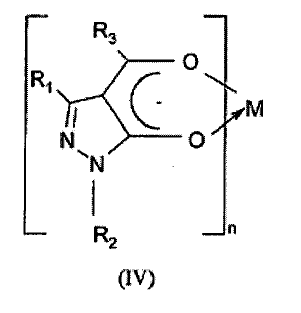 dans laquelle
M est un métal ;
n est la valence de M ;
R₁, R₂ et R₃ qui peuvent être les mêmes ou différents sont choisis parmi le groupe constitué par hydrogène, des groupes hydrocarbyle, des groupes aliphatiques substitués et non substitués et des structures de cycle aromatiques, hétérocycliques et polycycliques substituées et non substituées, des fluorocarbones, des halogènes tels que fluor ou des groupes thiophényle ou nitrile ; R₁ et R₃ peuvent également former des structures de cycle et R₁, R₂ et R₃ peuvent être copolymérisables avec un monomère, par exemple styrène ; et
des composés de la formule 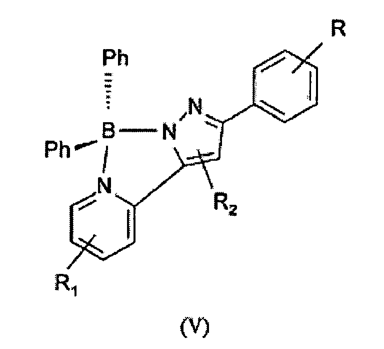 où Ph est un groupe phényle non substitué ou substitué où les substituants peuvent être les mêmes ou différents et sont choisis parmi hydrogène, des groupes aliphatiques substitués et non substitués, des structures de cycle aromatiques, hétérocycliques et polycycliques substituées et non substituées, fluorocarbone, des halogènes tels que fluor ou des groupes thiophényle et R, R₁ et R₂ peuvent être hydrogène ou des structures de cycle aromatiques, hétérocycliques et polycycliques substituées ou non substituées, fluor, des fluorocarbones, des halogènes tels que fluor ou des groupes thiophényle ou nitrile.

4. Composition de la revendication 1 ou 2, dans laquelle l'hôte comprend

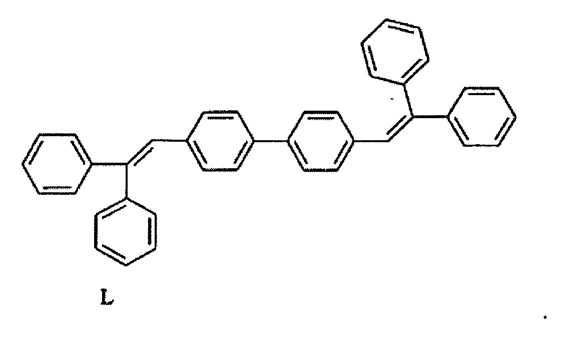

5. Dispositif électroluminescent qui comprend (i) une première électrode, (ii) une couche de composition électroluminescente comme revendiqué selon l'une quelconque des revendications 1 à 3 et (iii) une seconde électrode.

6. Dispositif électroluminescent selon la revendication 5 dans lequel la couche de la composition électroluminescente est un film d'une épaisseur inférieure à 1 µm.

7. Dispositif électroluminescent selon la revendication 5 ou 6 dans lequel il y a une couche en un matériau transport de trous entre la première électrode et la couche électroluminescente, le matériau transport de trous étant choisi parmi le groupe constitué par:
α-NPB, TPD, mTADATA ou un autre amine polyaromatique ;
un film en un polymère choisi parmi poly(vinylcarbazole), polyaniline, des polyanilines substitués, des polythiophènes, des polythiophènes substitués, des polysilanes et des polysilanes substitués ;
un copolymère d'aniline, un copolymère d'aniline avec o-anisidine, acide m-sulphanilique ou o-aminophénol, ou o-toluidine avec o-aminophénol, o-éthylaniline, o-phénylènediamine ou avec un aminoanthracène; et
un polymère conjugué choisi parmi poly(p-phénylènevinylène)-PPV et des copolymères incluant PPV, poly(2,5 dialcoxyphénylène vinylène), poly(2-méthoxy-5-(2-méthoxypentyloxy-1,4-phénylène vinylène), poly(2-méthoxypentyloxy)-1,4-phénylènevinylène), poly(2-méthoxy-5-(2-dodécyloxy-1,4-phénylènevinylène) et d'autres poly-(2,5-dialcoxyphénylènevinylènes), au moins l'un des groupes alcoxy étant un groupe alcoxy de solubilisation en chaîne longue, des polyfluorènes et des oligofluorènes, des polyphénylènes et des oligophénylènes, des polyanthracènes et des oligoanthracènes, des polythiophènes et des oligothiophènes.

8. Dispositif selon l'une quelconque des revendications 5-7, dans lequel il y a une couche en un matériau transport d'électrons entre la cathode et la couche de composé électroluminescent, la couche transport d'électrons étant choisie parmi le groupe constitué par :
un quinolate de métal ou un thioxinate de métal ;
quinolate d'aluminium, quinolate de zirconium ou quinolate de lithium, thioxinate de zinc, thioxinate de cadmium, thioxinate de gallium ou thioxinate d'indium ;
un matériau de formule Mx(DBM)n où Mx est un métal et DBM est dibenzoyl-méthane et n est la valence de Mx ; et
un cyanoanthracène tel que 9,10-dicyanoanthracène, un polystyrène sulphonate; et
Bebq, ZnPBO, ZnPBT, DTVb1, BND, OXD-7, TAZ et OXD-Star.
